# EUROPEAN PATENT APPLICATION

(11) **EP 1 466 576 A1**
(43) Date of publication of application: **13.10.2004**
(21) Application number: 03252311.0
(22) Date of filing: 11.04.2003
(51) Int. Cl.: A61H 39/00, A61N 1/36

(54) **Method and apparatus for using a varying frequency signal to stimulate the human body**

(71) Applicant: Chen, Yi-Ying, Vancouver, British Columbia (CA)
(72) Inventor: Wang, Wei-Cheng, Panchiao City, Taipei Heient, Taiwan (CN)
(74) Representative: Neobard, William John

(57) **Abstract**

A method and an apparatus for using a varying frequency signal to stimulate the human body (50) has a signal apparatus (10), a signal intensity controller (20) and an impedance matching unit (40). The signal apparatus (10) provides a varying frequency signal and outputs a voltage. The signal intensity controller (20) controls the intensity of the signal and the impedance matching unit (40) outputs the signal as an electric current to stimulate the human body (50). The varying frequency signal can overcome the adaptability of the human body (50) and the neurons to fixed stimulants. The present invention can improve the therapeutic effect and the varying frequency signal could be music.

## Description

### 1. Field of the Invention

The present invention relates to a method and an apparatus for using a varying frequency signal, and more particularly to a method and an apparatus for using varying frequency electric signals to stimulate a human body.

### 2. Description of Related Art

In general, acupuncture is a traditional medical science based on ancestral knowledge and experience in three oriental countries (Korea, China and Japan). In these countries in the past, the first step taken to cure an illness was to use acupuncture needle stimulation, and if it didn't work, the second step was medication. The order of these techniques shows how deeply our ancestors depended on acupuncture therapy.

However, traditional acupuncture techniques are not used as extensively in today's modem society. Recently, acupuncture therapy has begun using magnetic needles and electronic beams as well as traditional acupuncture needles.

As one has well known to those skilled in the art, the use of stimulating electric signals is the prevalent means of practicing acupuncture. In general, the stimulating electric signals have a number of physical effects on a human body including a cell effect, a tissue effect and a generalized effect. The cell effect includes exciting peripheral neurons, changing non-excited cell permeability and so on. The tissue effect includes skeletal muscle contraction, smooth muscle contraction, influencing blood volume in blood vessels and improving tissue regeneration. The generalized effect includes influencing the endocrine and neurotransmitters to act as an anesthesia.

A related art uses fixed frequency signals to stimulate the human body. Because of the frequency, voltage and current used as stimulation are fixed, the adaptability of the human body and the sensory neuron system may develop a tolerance or adaptation to the stimulation. The so-called adaptability of the human body and the sensory neuron system means that, when the stimulation of the neuron is fixed (of same strength and of same frequency), the response of neuron to the stimuli becomes retarded (the decreases of sensibility of sensory). This is because the elevation of threshold of action potential of responding sensory neuron or acting muscles group. When a fixed signal stimulates the neuron over a long period of time, the fixed signal loses its ability to the effect the stimulation. For example, the sensory neuron becomes tolerant to the stimulation because of elevation of threshold of generating the action potential. For the muscular contraction, when using fixed stimuli, the elevation of threshold generates action potential that leads to the retardation of muscular contraction. These phenomena weaken the effect of stimuli. Therefore, after a worth of three to five minutes stimulation, the human body sensory neuron system produces the adaptation and to reject the stimuli. The same adaptation process occurs to the muscular contraction. Almost every type of fix stimulation can result in the problem associated with human adaptability.

The present invention has arisen to mitigate or obviate the disadvantages of the method and apparatus for using fixed frequency signals to stimulate the human body.

The main objective of the present invention is to overcome the adaptability of the muscular contraction process and the neuronal sensory system by providing varying frequency signals to stimulate the target area of human body that incorporates an audio stimuli.

To achieve the objective, a method and an apparatus for using varying frequency signals to stimulate human body comprises a signal apparatus, a signal intensity controller and an impedance matching unit. The signal apparatus provides a varying frequency signal. The varying frequency signal is an audio signal. The signal intensity controller controls the intensity of the signal from the signal apparatus. The impedance matching unit is arranged between the signal intensity controller and a human body. The impedance matching unit changes the signal to electric current to stimulate the human body. A filter and signal amplification apparatus can be mounted between the signal intensity controller and the impedance matching unit. The filter and signal amplification apparatus filters the undesired frequency bandwidth of the signal and amplifies the intensity of the signal used.

Further benefits and advantages of the present invention will become apparent after a careful reading of the detailed description with appropriate reference to the accompanying drawings.

### IN THE DRAWINGS

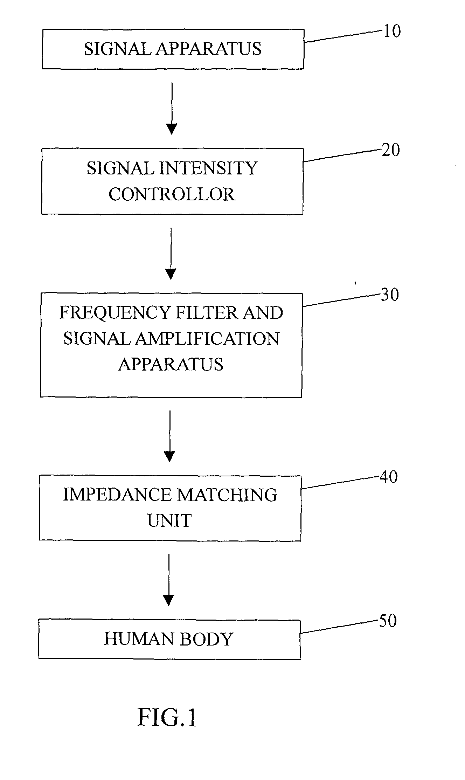

Fig. 1 is a schematic block diagram of the apparatus in accordance with the present invention.

With reference to Fig.1, an apparatus for using varying frequency signals to stimulate a human body for purposes of acupuncture treatment comprises a signal apparatus (10), a signal intensity controller (20) and an impedance matching unit (40). The signal apparatus (10) provides a varying frequency signal. The varying frequency signal is an audio signal and a voltage output. The voltage intensity is controlled by the audio signal intensity. The signal intensity controller (20) is connected to the signal apparatus (10). The signal intensity controller (20) adjusts and controls the intensity of the voltage of the signal received from the signal apparatus (10). The impedance matching unit (40) is mounted between the signal intensity controller (20) and a human body (50). The impedance matching unit (40) changes the voltage to an electric current to stimulate the human body. If the user wants a specific frequency bandwidth or the audio signal is too weak, a frequency filter and signal amplification apparatus (30) is mounted between the signal intensity controller (20) and the impedance matching unit (40). The frequency filter and signal amplification apparatus (30) filters undesired frequency bandwidth and amplifies the intensity of the signal used.

A method and an apparatus for using a varying frequency signal to stimulate the human body in accordance with the present invention uses music as the varying frequency signal. Most contemporary and classical music consists of widely varying tonal or frequency signals, so music as a source of signals to stimulate a human body will not easily cause the human body and the neuron to develop a tolerance and adapt to the signal. The range of the music frequency used is range from 20Hz to 10KHz. Furthermore, Chinese medical research shows the audio stimuli of irregular noise, music and language output as electric stimuli have beneficial clinical effects, for example they obviously relieved pain, relaxed patients and changed the blood circulation. Using varying frequency signals to stimulate the human body may be a good method to provide better treatment of the human body.

The music is the source of the varying frequency electric signals and at the same time provides audio stimuli. The audio stimuli and the electric stimuli to the skin are used together to make the better treatment. The electric stimuli stimulates the skin to relax the body and neurons, the music as audio stimuli also reduces rigidity in a tired body. Music as audio stimuli functions to relax the mind and the body. Theoretically speaking, the human body will accept the simultaneous application of the audio stimuli and the electric stimuli.

A method for using varying frequency signals to stimulate human body in accordance with the present invention comprises the following steps. Receiving a varying frequency signal and outputting a voltage. Adjusting the intensity of the varying frequency signal for each person. If the varying frequency signal is too weak or the user wants a specific frequency bandwidth, a frequency filter and signal amplification apparatus can filter unused frequency bandwidth and amplify the intensity of the remaining varying frequency signal. As the last step, the varying frequency signal is passed in a human body as an electric current through an electrode. The electrode attaches to the human body so the current will stimulate the human body. The electrode may be an electrode probe or pad or an electrode needle.

The advantage of the invention is that the varying frequency signal not only stimulate the human body by electric stimuli but also provides music as an audio stimuli. The varying frequency signal can overcome the adaptability of the human body and the neurons to fixed stimulants, and the music provides an additional audio stimulus. The present invention can improve the therapeutic effect.

Although the invention has been explained in relation to its preferred embodiment, it is to be understood that many other possible modifications and variations can be made without departing from the spirit and scope of the invention as hereinafter claimed.

## Claims

1. An apparatus for using a varying frequency signal to stimulate the human body comprising:
a signal apparatus (10) providing a varying frequency signal,
a signal intensity controller (20) connected to the signal apparatus (10) for adjusting and controlling the intensity of the varying frequency signal received from the signal apparatus (10); and
an impedance matching unit (40) mounted to the signal intensity controller (20) to output the frequency signals as an electric current to stimulate a human body (50).

2. The apparatus for using a varying frequency signal to stimulate the human body as claimed in claim 1, wherein a frequency filter and signal amplification apparatus (30) is mounted between the signal intensity controller (20) and the impedance matching unit (40) to filter undesired frequencies and to amplify the signal.

3. The apparatus for using a varying frequency signal to stimulate the human body as claimed in claim 1, wherein the varying frequency signal received in the signal apparatus (10) is music.

4. The apparatus for using a varying frequency signal to stimulate the human body as claimed in claim 3, wherein the range of the varying frequency signal in the signal apparatus (10) is 20Hz to 10KHz.

5. A method for using a varying frequency signal to stimulate the human body comprising:
receiving a varying frequency signal;
adjusting and controlling the intensity of the varying frequency signal for each person;
outputting the varying frequency signal output as an electric current through an electrode; and
applying the electric current from the electrode to a human body to stimulate the human body.

6. The method for using a varying frequency signal to stimulate the human body as claimed in claim 5, wherein the range of the varying frequency signal is 20Hz to 10KHz.

7. The method for using a varying frequency signal to stimulate the human body as claimed in claim 5, wherein the intensity of the signal is amplified by a frequency filter and signal amplification apparatus (30).

8. The method for using a varying frequency signal to stimulate the human body as claimed in claim 5, wherein the frequency variation signal is music.

9. The method for using a varying frequency signal to stimulate the human body as claimed in claim 8, wherein the music is output also as an audio signal.

10. The method for using a varying frequency signal to stimulate the human body as claimed in claim 5, wherein the electrode is one of an electrode probe, an electrode pad and an electrode needle.
